# NEUE EUROPÄISCHE PATENTSCHRIFT

(11) **EP 1 286 963 B2**
(45) Veröffentlichungstag und Bekanntmachung des Hinweises auf die Entscheidung über den Einspruch: **24.10.2012**
(45) Hinweis auf die Patenterteilung: 10.12.2003
(21) Anmeldenummer: 01936425.6
(22) Anmeldetag: 02.06.2001
(51) Int. Cl.: C07D 201/16

(54) **VERFAHREN ZUR ABTRENNUNG VON AMMONIAK AUS LÖSUNGEN, DIE CAPROLACTAM UND AMMONIAK ENTHALTEN**
METHOD FOR SEPARATING AMMONIA FROM SOLUTIONS CONTAINING CAPROLACTAM AND AMMONIA
VERFAHREN ZUR ABTRENNUNG VON AMMONIAK AUS LOSUNGEN, DIE CAPROLACTAM UND AMMONIAK ENTHALTEN

(30) Priorität: 05.06.2000 DE 10027328; 11.07.2000 DE 10033516
(43) Veröffentlichungstag der Anmeldung: 05.03.2003
(73) Patentinhaber: BASF SE, 67056 Ludwigshafen (DE)
(72) Erfinder: LUYKEN, Hermann, 67069 Ludwigshafen (DE); OHLBACH, Frank, 40219 Düsseldorf (DE); MAIXNER, Stefan, 68723 Schwetzingen (DE); FISCHER, Rolf-Hartmuth, 69121 Heidelberg (DE); MELDER, Johann-Peter, 67459 Böhl-Iggelheim (DE); BASSLER, Peter, 68519 Viernheim (DE); ANSMANN, Andreas, 69168 Wiesloch (DE)
(86) Internationale Anmeldenummer: PCT/EP2001/006310
(87) Internationale Veröffentlichungsnummer: WO 2001/094308

(56) Entgegenhaltungen:
- DE-A- 19 500 222
- DE-A- 19 548 289

## Beschreibung

Die vorliegende Erfindung betrifft ein Verfahren zur destillativen Abtrennung von Ammoniak aus Lösungen (I), die ein Lactam und Ammoniak enthalten, dadurch gekennzeichnet, dass die Abtrennung bei einem Absolutdruck von weniger als 10 bar in einer Destillationsvorrichtung (a) durchgeführt wird, wobei am Kopf der zur Abtrennung eingesetzten Destillationsvorrichtung (a) zur Abtrennung von Ammoniak aus Lösung (I) ein Kondensator (ak) vorhanden ist, in dem höher als Ammoniak siedende Komponenten teilweise oder vollständig kondensiert werden unter Erhalt eines Kondensats (III), wobei das Kondensat (III) aus dem Kondensator (ak) in die Trennvorrichtung (a) zurückgeführt wird.

Lösungen, die ein Lactam und Ammoniak enthalten, fallen bei zahlreichen chemischen Verfahren an, beispielsweise bei der Herstellung cyclischer Lactame durch Umsetzung von omega-Aminocarbonsäurederivaten mit Wasser.

Die Aufarbeitung dieser Lösungen ist problematisch, da üblicherweise an die Lactame, insbesondere an Caprolactam, bekannterweise extrem hohe Reinheitsanforderungen gestellt werden.

So beschreiben WO 95/14665 und WO 95/14664 die Umsetzung von 6-Aminocapronitril ("ACN") in der Flüssigphase mit Wasser in Gegenwart von heterogenen Katalysatoren und einem Lösungsmittel zu einer Lösung enthaltend Caprolactam und Ammoniak. Eine Auf arbeitung dieser Lösung ist nicht beschrieben.

In der Regel wird bei der destillativen Abtrennung von Ammoniak aus Ammoniak enthaltenden Lösungen ein hoher Druck, üblicherweise von höher als 14 bar absolut, angestrebt, um eine geeignete Temperatur bei der Kondensation von Ammoniak am Kopf der Destillationsvorrichtung zu erhalten.

Der vorliegenden Erfindung lag die Aufgabe zugrunde, ein Verfahren bereitzustellen, das die Abtrennung des Ammoniaks aus Lösungen, die ein Lactam und Ammoniak enthalten, auf technisch einfache und wirtschaftliche Weise ermöglicht.

Demgemäß wurde das eingangs definierte Verfahren gefunden.

Erf indungsgemäß enthält Lösung (I) ein Lactam und Ammoniak.

Prinzipiell sind keine Begrenzungen hinsichtlich des Lactams bekannt. Bevorzugt kommen Lactame von C₄-C₂₀-omega-Carbonsäuren in Betracht, wie 4-Aminobutansäurelactam, 5-Aminiopentansäurelactam, 6-Aminohexansäurelactam ("Caprolactam"), 7-Aminoheptansäurelactam oder 8-Aminooctansäurelactam, besonders bevorzugt Caprolactam.

Diese Lactame können substituiert sein, beispielweise durch C1-C4-Alkylgruppen, Halogene, wie Fluor, Chlor oder Brom, C1-C4-Alkoxygruppen oder C1-C4-Carboxygruppen, vorzugsweise sind die Lactame nicht substituiert.

Es können auch Mischungen solcher Lactame eingesetzt werden.

Solche Lactame sind an sich bekannt.

Die Herstellung solcher Lactame kann durch Umsetzung der entsprechenden Aminonitrile mit Wasser erfolgen, beispielsweise im Falle von Caprolactam durch Umsetzung von 6-Aminocapronitril, wie zum Beispiel in EP-A-0 659 741, WO 95/14664, WO 95/14665, WO 96/22874, WO 96/22974, WO 97/23454, WO 99/28296 oder WO 99/47500 beschrieben.

Bei einer solchen Umsetzung erhält der Reaktionsaustrag mindestens ein mol Ammoniak pro mol Lactam. Daneben kann der Reaktionsaustrag weitere Komponenten, wie nicht umgesetztes Aminonitril, überschüssiges oder im Falle einer Umsetzung in der Gasphase zum Quenchen des Reaktionsaustrags eingesetztes Wasser oder gegebenenf alls organische Lösungsmittel enthalten.

Im erfindungsgemäßen Verfahren können als Lösung (I) jede Mischung aus einem Lactam und Ammoniak, vorteilhaft einer der genannten Reaktionsausträge, oder Gemische solcher Systeme eingesetzt werden.

Erfindungsgemäß destilliert man Ammoniak aus Lösung (I) bei einem Druck von weniger als 10 bar absolut, vorzugsweise weniger als 8 bar absolut, ab. Dabei kommt vorteilhaft ein Druck oberhalb des atmosphärischen Umgebungsdrucks in Betracht.

Destillationsdruck und Destillationstemperatur sollten vorzugsweise so gewählt werden, daß über Kopf ein im wesentlichen Ammoniak enthaltender Strom gasförmig abgezogen werden kann.

Als Destillationsvorrichtung (a) kommen hierfür übliche einstufige oder mehrstufige Apparaturen in Betracht, wie sie beispielsweise in: Kirk-Othmer, Encyclopedia of Chemical Technology, 3. Ed., Vol. 7, John Wiley & Sons, New York, 1979, Seite 870-881 beschrieben sind, wie Verdampfungsblasen oder Rektifikationskolonnen, beispielsweise Siebbodenkolonnen, Glockenbodenkolonnen, Pakkungskolonnen oder Füllkörperkolonnen.

Hierbei können einstufige Destillationsblasen, reine Abtriebskolonnen oder Rektifikationskolonnen mit Abtriebs- und Verstärkungsteil verwendet werden.

In einer bevorzugten Ausführungsform kann der am Kopf der Destillationsvorrichtung (a) erhaltene dampfförmige, im wesentlichen Ammoniak enthaltende Strom durch einen Kondensator (ak) geleitet werden. In diesem Kondensator können vorteilhaft höher als Ammoniak siedende Verbindungen teilweise oder vollständig auskondensiert werden unter Erhalt eines Kondensats (III) und einer im wesentlichen Ammoniak enthaltenden Gasphase.

Kondensat (III) kann vorteilhaft teilweise oder vollständig in die Destillationsvorrichtung (a) zurückgeführt werden, insbesondere für den Fall, daß als Destillationsvorrichtung (a) eine Kolonne verwendet wird, besonders bevorzugt, wenn diese Kolonne einen Verstärkungsteil enthält.

Aus dem im wesentlichen Ammoniak enthaltenden Strom aus Vorrichtung (a) oder Kondensator (ak) kann Ammoniak teilweise oder vollständig durch Absorption in einem flüssigen Lösungsmittel (II) kondensiert werden. Der Strom kann dazu vorher verdichtet werden.

Als Absorptionsvorrichtung (b) kommen hierfür übliche einstufige oder mehrstufige Apparaturen in Betracht, wie sie beispielsweise in: Kirk-Othmer, Encyclopedia of Chemical Technology, 3. Ed., Vol. 7, John Wiley & Sons, New York, 1979, Seite 870-881 beschrieben sind, wie Blasensäulen oder Absorptionskolonnen, beispielsweise Siebbodenkolonnen, Glockenbodenkolonnen, Packungskolonnen oder Füllkörperkolonnen.

Es kann vorteilhaft sein, zwischen den Trennstufen der Absorptionskolonne (b) eine Kühlung durchzuführen.

In einer bevorzugten Ausführungsform kann Kolonne (a) mit einem Verstärkungsteil versehen werden, der mit einem Lösungsmittel (IV) als Rücklauf betrieben wird. Vorzugsweise sollte das Lösungsmittel (IV) im wesentlichen ammoniakfrei sein. Vorteilhaft kann als Rücklauf ein Teil des in der Absorption mit Ammoniak beladenen Lösungsmittel (II) verwendet werden.

Vorteilhaft kommen als Lösungsmittel (II), Lösungsmittel (IV) oder beiden Lösungsmitteln Wasser oder, im Falle der Herstellung des Lactams aus dem entsprechenden Aminonitril, das bei dieser Umsetzung eingesetzte Lösungsmittel in Betracht.

In einer bevorzugten Ausführungsform kann aus dem Kolonne (b) beladenen Lösungsmittel (II) Ammoniak durch Destillation oder Rektifikation in einer Destillationsvorrichtung (c) abgetrennt werden.

Als Destillationsvorrichtung (c) kommen hierfür übliche einstufige oder mehrstufige Apparaturen in Betracht, wie sie beispielsweise in: Kirk-Othmer, Encyclopedia of Chemical Technology, 3. Ed., Vol. 7, John Wiley & Sons, New York, 1979, Seite 870-881 beschrieben sind, wie Verdampfungsblasen oder Rektifikationskolonnen, beispielsweise Siebbodenkolonnen, Glockenbodenkolonnen, Pakkungskolonnen oder Füllkörperkolonnen.

Hierbei können einstufige Destillationsblasen, reine Abtriebskolonnen oder Rektifikationskolonnen mit Abtriebs- und Verstärkungsteil verwendet werden.

Ein Teil des kondensierten Stroms kann vorteilhaf t als Rücklauf auf Kolonne (a) zurückgefahren werden. Dabei kommt eine Entspannungsverdampfung mit entsprechender Abkühlung des Rücklaufstroms in Betracht.

Für eine Verdichtung kommen insbesondere solche Drücke in Betracht, unter denen Ammoniak im Temperaturbereich von 0 bis 50°C flüssig vorliegt. Solche Temperaturen können beispielsweise durch Kühlung mit Flußwasser oder Luft auf technisch einfache Weise eingestellt werden.

Für die Verdichtung können an sich bekannte Vorrichtungen, wie Turbo-, Kolben-, Membran- oder vorzugsweise Flüssigkeitsringverdichter eingesetzt werden.

In einer bevorzugten Variante kann die Verdichtung mit einem Flüssigkeitsringverdichter durchgeführt werden, wobei dieser mit Lösungsmittel (II), insbesondere ammoniakbeladenem Lösungsmittel (II) aus der Absorption, beschickt werden kann.

Die überlauf ende Flüssigkeit aus dem Flüssigkeitsringverdichter kann teilweise oder vollständig als Rücklauf auf Kolonne (a) zurückgefahren werden.

Das erfindungsgemäße Verfahren hat den Vorteil, daß die Entstehung von Feststoffen bei der destillativen Abtrennung von Ammoniak aus Lactam, insbesondere Caprolactam, und Ammoniak enthaltenden Lösungen, die aus der Herstellung von Lactam durch Umsetzung von Aminonitril mit Wasser stammen, in der Destillationskolonne, die einen kontinuierlichen Betrieb der Aufarbeitung hindern, vermindert wird.

### Vergleichsbeispiel

100 kg/h ACN wurden in 1 000 kg/h Ethanol zu Caprolactam umgesetzt, wobei die Umsetzung an einem oxidischen Katalysator in flüssiger Phase erfolgte. Der Reaktionsaustrag enthielt 12 Gew.-% Ammoniak und wurde zur Destillation in eine Siebbodenkolonne geleitet, die 20 Siebböden enthielt. Am Kopf der Kolonne war ein Kondensator vorhanden. Die Kolonne wurde bei einem Druck von 15 bar absolut betrieben. Der als Kondensat aus dem Kondensator anf allende flüssige Ammoniak wurde zum Teil als Rücklauf auf den Kolonnenkopf zurückgefahren, die Rücklaufmenge betrug 200 kg/h. Der Ammoniakgehalt im über Sumpf abgezogenen Strom betrug weniger als 1 000 ppm. 30 h nachdem die Kolonne angefahren wurde, kam es zu Verstopfungen der Siebböden, daran erkennbar, dass die Druckdifferenz zwischen Sumpf und Kopf bei gleichbleibender Belastung der Kolonne drastisch anstieg. Die Kolonne musste abgestellt und mit Wasser gespült werden.

### Beispiel

Der flüssige Reaktionsaustrag aus dem Vergleichsbeispiel wurde in die gleiche Kolonne geleitet, diese aber bei einem Druck von 5 bar absolut betrieben. Der Brüdenstrom aus dem Kopf der Kolonne wurde durch den Kondensator geleitet, jedoch nicht sämtlicher Ammoniak auskondensiert. Der verbleibende dampfförmige Ammoniak wurde abgezogen und die als Kondensat anfallende Flüssigkeit als Rücklauf auf die Kolonne zurückgefahren. Die Kolonne konnte über mehrere Wochen stabil betrieben werden.

## Patentansprüche

1. Verfahren zur destillativen Abtrennung von Ammoniak aus Lösungen (I), die ein Lactam und Ammoniak enthalten, **dadurch gekennzeichnet, dass** die Abtrennung bei einem Absolutdruck von weniger als 10 bar in einer Destillationsvorrichtung (a) durchgeführt wird, wobei am Kopf der zur Abtrennung eingesetzten Destillationsvorrichtung (a) zur Abtrennung von Ammoniak aus Lösung (I) ein Kondensator (ak) vorhanden ist, in dem höher als Ammoniak siedende Komponenten teilweise oder vollständig kondensiert werden unter Erhalt eines Kondensats (III), wobei das Kondensat (III) aus dem Kondensator (ak) in die Trennvorrichtung (a) zurückgeführt wird.

2. Verfahren nach Anspruch 1, wobei man die Abtrennung bei einem Absolutdruck von weniger als 8 bar durchführt.

3. Verfahren nach Anspruch 1 oder 2, wobei über Kopf ein im wesentlichen Ammoniak enthaltender Strom dampfförmig abgezogen wird.

4. Verfahren nach den Ansprüchen 1 bis 3, wobei der dampfförmige Strom aus Destillationsvorrichtung (a) oder Kondensator (ak), enthaltend im wesentlichen Ammoniak, durch Absorption in einem flüssigen Lösungsmittel (II) kondensiert wird.

5. Verfahren nach Anspruch 4, wobei die Absorption in einer Absorptionsvorrichtung (b) stattfindet.

6. Verfahren nach Anspruch 5, wobei zwischen den Trennstufen der Absorptionsvorrichtung (b) eine Kühlung stattfindet.

7. Verfahren nach den Ansprüchen 1 bis 6, wobei die Destillationsvorrichtung (a) zur Abtrennung von Ammoniak aus Lösung (I) einen Verstärkungsteil enthält, der mit einem Lösungsmittel (IV) als Rücklauf betrieben wird.

8. Verfahren nach Anspruch 7, wobei das Lösungsmittel (IV) im wesentlichen ammoniakfrei ist.

9. Verfahren nach Anspruch 7, wobei als Rücklauf ein Teil des in der Absorption mit Ammoniak beladenen Lösungsmittels (II) verwendet wird.

10. Verfahren nach den Ansprüchen 1 bis 9, wobei als Lösungsmittel (II) oder als Lösungsmittel (IV) Wasser verwendet wird.

11. Verfahren nach den Ansprüchen 1 bis 10, wobei als Lösungsmittel (II) oder als Lösungsmittel (IV) das gleiche Lösungsmittel wie bei der Herstellung des Lactams aus dem entsprechenden Aminonitril verwendet wird.

12. Verfahren nach den Ansprüchen 1 bis 11, wobei Ammoniak aus dem beladenen Lösungsmittel (II) durch Destillation oder Rektifikation in einer Destillationsvorrichtung (c) abgetrennt wird.

13. Verfahren nach Anspruch 12, wobei statt Destillationsvorrichtung (c) eine einstufige Verdampfung verwendet wird.

14. Verfahren nach den Ansprüchen 1 bis 3, wobei der dampfförmige Strom aus Kondensator (ak), enthaltend im wesentlichen Ammoniak, verdichtet und teilweise oder vollständig kondensiert wird.

15. Verfahren nach Anspruch 14, wobei ein Teil des kondensierten Stromes als Rücklauf auf Destillationsvorrichtung (a) zurückgefahren wird, wobei es zu einer Entspannungsverdampfung mit entsprechender Abkühlung des Rücklaufstromes kommt.

16. Verfahren nach den Ansprüchen 1 bis 3, wobei der dampfförmige Strom aus Destillationsvorrichtung (a) oder Kondensator (ak), enthaltend im wesentlichen Ammoniak, verdichtet und anschließend nach den Ansprüchen 4 bis 13 verfahren wird.

17. Verfahren nach den Ansprüchen 14 bis 16, wobei die Verdichtung des im wesentlichen Ammoniak enthaltenden Stroms mit einem Flüssigkeitsringverdichter erfolgt.

18. Verfahren nach Anspruch 17, wobei der Flüssigkeitsringverdichter mit Lösungsmittel (II) beschickt wird.

19. Verfahren nach den Ansprüchen 1 bis 18, wobei man als Lactam Caprolactam einsetzt.

## Claims

1. The process for distillative removal of ammonia from solutions (I) which comprise a lactam and ammonia, which comprises effecting said removal in a distillation apparatus (a) at an absolute pressure of less than 10 bar, wherein said distillation apparatus (a) is equipped at the top with a condenser (ak) in which components having a boiling point higher than that of ammonia are partly or completely condensed to obtain a condensate (III), wherein said condensate (III) is recycled from said condenser (ak) into the separator (a).

2. The process according to claim 1, wherein said removal is effected at an absolute pressure of less than 8 bar.

3. The process according to claim 1 or 2, wherein an essentially ammonia-comprising stream is taken off overhead in vaporous form.

4. The process according to any of claims 1 to 3, wherein said vaporous essentially ammonia-comprising stream from said distillation apparatus (a) or said condenser (ak) is condensed by absorption in a liquid solvent (II).

5. The process according to claim 4, wherein said absorption takes place in an absorber (b).

6. The process according to claim 5, wherein cooling takes place between the separating stages of said absorber (b).

7. The process according to any of claims 1 to 6, wherein said distillation apparatus (a) comprises a rectifying section operated with a solvent (IV) as reflux.

8. The process according to claim 7, wherein said solvent (IV) is essentially ammonia free.

9. The process according to claim 7, wherein a portion of said solvent (II) loaded with ammonia in said absorption is used as reflux.

10. The process according to any of claims 1 to 9, wherein said solvent (II) or said solvent (IV) is water.

11. The process according to any of claims 1 to 10, wherein said solvent (II) or said solvent (IV) is the same solvent as used in the production of the lactam from the corresponding aminonitrile.

12. The process according to any of claims 1 to 11, wherein ammonia is removed from said loaded solvent (II) by distillation or rectification in a distillation apparatus (c).

13. The process according to claim 12, wherein a single evaporation stage is used instead of said distillation apparatus (c).

14. The process according to any of claims 1 to 3, wherein said vaporous essentially ammonia-comprising stream from said condenser (ak) is compressed and partly or completely condensed.

15. The process according to claim 14, wherein a portion of said condensed stream is recycled as reflux into said distillation apparatus (a) for a flash evaporation with corresponding cooling of the reflux stream.

16. The process according to any of claims 1 to 3, wherein said vaporous essentially ammonia-comprising stream from said distillation apparatus (a) or said condenser (ak) is compressed and subsequently processed according to any of claims 4 to 13.

17. The process according to any of claims 14 to 16, wherein said compressing of said essentially ammonia-comprising stream is effected using a liquid ring compressor.

18. The process according to claim 17, wherein said liquid ring compressor is charged with said solvent (II).

19. The process according to any of claims 1 to 18, wherein said lactam is caprolactam.

## Revendications

1. Procédé pour la séparation d'ammoniac par distillation à partir de solutions (I) qui contiennent un lactame et de l'ammoniac, **caractérisé en ce qu'**on effectue la séparation sous une pression absolue de moins de 10 bars dans un dispositif de distillation (a), à la tête du dispositif de distillation (a) utilisé pour la séparation étant présent pour la séparation d'ammoniac d'avec la solution (I) un condenseur (ak) dans lequel les composants à plus haut point d'ébullition que l'ammoniac sont partiellement ou totalement condensés, avec obtention d'un condensat (III), le condensat (III) provenant du condenseur (ak) étant renvoyé dans le dispositif de séparation (a).

2. Procédé selon la revendication 1, dans lequel on effectue la séparation sous une pression absolue de moins de 8 bars.

3. Procédé selon la revendication 1 ou 2, dans lequel un courant contenant essentiellement de l'ammoniac est évacué sous forme de vapeur par la tête.

4. Procédé selon les revendications 1 à 3, dans lequel le courant sous forme de vapeur provenant du dispositif de distillation (a) ou du condenseur (ak), contenant essentiellement de l'ammoniac, est condensé par absorption dans un solvant liquide (II).

5. Procédé selon la revendication 4, dans lequel l'absorption a lieu dans un dispositif d'absorption (b).

6. Procédé selon la revendication 5, dans lequel un refroidissement a lieu entre les stades de séparation du dispositif d'absorption (b).

7. Procédé selon les revendications 1 à 6, dans lequel le dispositif de distillation (a) pour la séparation d'ammoniac à partir de la solution (I) comporte un élément d'enrichissement que l'on fait fonctionner avec un solvant (IV) en tant que reflux.

8. Procédé selon la revendication 7, dans lequel le solvant (IV) est essentiellement exempt d'ammoniac.

9. Procédé selon la revendication 7, dans lequel on utilise comme reflux une partie du solvant (II) chargé d'ammoniac dans l'absorption.

10. Procédé selon les revendications 1 à 9, dans lequel on utilise de l'eau comme solvant (II) ou comme solvant (IV).

11. Procédé selon les revendications 1 à 10, dans lequel on utilise comme solvant (II) ou comme solvant (IV) le même solvant que dans la production du lactame à partir de l'aminonitrile correspondant.

12. Procédé selon les revendications 1 à 11, dans lequel l'ammoniac est séparé du solvant chargé (II) par distillation ou rectification dans un dispositif de distillation (c).

13. Procédé selon la revendication 12, dans lequel au lieu d'un dispositif de distillation (c) on utilise une vaporisation en une étape.

14. Procédé selon les revendications 1 à 3, dans lequel le courant sous forme de vapeur provenant du condenseur (ak), contenant essentiellement de l'ammoniac, est comprimé et partiellement ou totalement condensé.

15. Procédé selon la revendication 14, dans lequel une partie du courant condensé est renvoyé en tant que reflux dans le dispositif de distillation (a), de sorte qu'il se produit une vaporisation par détente, avec refroidissement correspondant du courant de reflux.

16. Procédé selon les revendications 1 à 3, dans lequel on comprime le courant sous forme de vapeur provenant du dispositif de distillation (a) ou du condenseur (ak), contenant essentiellement de l'ammoniac, et on procède ensuite conformément aux revendications 4 à 13.

17. Procédé selon les revendications 14 à 16, dans lequel la compression du courant contenant essentiellement de l'ammoniac s'effectue au moyen d'un compresseur à anneau liquide.

18. Procédé selon la revendication 17, dans lequel on alimente en solvant (II) le compresseur à anneau liquide.

19. Procédé selon les revendications 1 à 18, dans lequel on utilise en tant que lactame du caprolactame.
